# EUROPÄISCHE PATENTSCHRIFT

(11) **EP 1 246 688 B1**
(45) Veröffentlichungstag und Bekanntmachung des Hinweises auf die Patenterteilung: **12.05.2004**
(21) Anmeldenummer: 01987692.9
(22) Anmeldetag: 18.10.2001
(51) Int. Cl.: B01D 71/10, B01D 71/16, B01D 69/12, B01D 69/14

(54) **SIEBDRUCKFÄHIGE PASTE ZUR HERSTELLUNG EINER PORÖSEN POLYMERMEMBRAN FÜR EINEN BIOSENSOR**
PASTE, WHICH CAN UNDERGO SCREEN PRINTING, FOR PRODUCING A POROUS POLYMER MEMBRANE FOR A BIOSENSOR
PATE POUVANT ETRE IMPRIMEE PAR SERIGRAPHIE, UTILISEE POUR PRODUIRE UNE MEMBRANE POLYMERE POREUSE POUR UN BIODETECTEUR

(30) Priorität: 19.10.2000 DE 10052066
(43) Veröffentlichungstag der Anmeldung: 09.10.2002
(73) Patentinhaber: Inverness Medical Limited, Iverness IV2 3ED (GB)
(72) Erfinder: STIENE, Matthias, Inverness IV2 3QG (GB); VON TIEDEMANN, Birgit, Inverness, IV2 3HN (GB); RODGERS, Jamie, Inverness IV2 4LL (GB); MACGREGOR, Lucy, Inverness IV2 3DL (GB); MCALEER, Jerry, Grove (Oxon) OX12 ONR (GB); MCNEILAGE, Alan, Inverness, IV3 8LZ (GB)
(74) Vertreter: Mercer, Christopher Paul
(86) Internationale Anmeldenummer: PCT/EP2001/012073
(87) Internationale Veröffentlichungsnummer: WO 2002/032559

(56) Entgegenhaltungen:
- US-A- 4 425 263
- US-A- 5 607 566
- US-A- 5 658 444

## Beschreibung

Die vorliegende Erfindung betrifft eine siebdruckfähige Paste zur Herstellung einer porösen Polymermembran, welche bei elektrochemischen Sensoren, insbesondere bei elektrochemischen Biosensoren, zur integrierten Vorbereitung von insbesondere Vollblutproben verwendet werden kann.

Biosensoren finden bereits in einer Vielzahl diagnostischer Verfahren, beispielsweise bei der Bestimmung der Konzentration verschiedener Faktoren in Körperflüssigleiten, wie dem Blut, Anwendung. Angestrebt werden dabei Sensoren, die keine aufwendige Aufarbeitung der (Blut-)Probe erfordern, sondern bereits durch bloßes Auftragen der Körperflüssigkeit auf einen Teststreifen ein schnelles Ergebnis liefern. Dabei läuft eine spezifische biochemische Reaktion ab, wie beispielsweise die enzymatische Umsetzung der zu bestimmenden Komponente, welche dann einen Elektronentransfer zwischen verschiedenen Elektroden (Arbeits- und Referenzelektroden) bewirkt, der quantitativ bestimmt werden kann.

Nachteilig an den meisten bekannten elektrochemischen Biosensoren ist, dass beim Auftragen des Blutes auf den dafür vorgesehenen Bereich des Teststreifens die ablaufende biochemische Reaktion durch andere, im Blut enthaltene Bestandteile, vor allem die roten Blutkörperchen (Erythrozyten), beeinflusst wird. So ist beispielsweise bei hohen Hämatokritwerten (= Volumenanteil der Erythrozyten an der gesamten Blutmenge in Vol.Gew.-%) der mit Hilfe von herkömmlichen Blutglucosesensoren gemessene Glucosewert niedriger als der tatsächliche Wert. Diese Beeinträchtigung entsteht dadurch, dass die Erythrozyten durch Adsorption an der reaktiven Schicht des Biosensors die Diffusion der Glucose in diese und zur Elektrode beeinflussen und das Messsignal verringern.

Zur Lösung dieses Problems wurden verschiedene Membranen vorgeschlagen, die über der auf den Elektroden angeordneten Enzymschicht des Teststreifens aufgebracht werden, um die Erythrozyten von dieser fernzuhalten.

So beschreibt beispielsweise das US-Patent 5,658,444 eine Erythrozytenausschlussmembran für einen Sensor, welche aus einem wasserunlöslichen, hydrophoben Polymeren, einem wasserlöslichen hydrophilen Polymeren und einem Erythrozytenaggregationsagens besteht und durch Aufsprühen auf die Oberfläche des Teststreifens hergestellt wird.

Nachteilig bei dieser Membran ist zum einen, dass der Porendurchmesser der Membran in Abhängigkeit der Sprühdistanz und des Aufsprühdruckes variiert. Außerdem bedeutet das Aufsprühen der Membran bei der Produktion des Teststreifens einen zusätzlichen, von der Herstellung des übrigen Teststreifens verschiedenen und deshalb aufwendigen Arbeitsgang, was den Produktionsvorgang verkompliziert und damit verteuert.

Aufgabe der vorliegenden Erfindung ist es deshalb, eine Paste zur Herstellung einer porösen Membran zur Verfügung zu stellen, welche die genannten Nachteile nicht aufweist, indem sie während des Herstellungsprozesses des Biosensors durch ein sich in den übrigen Ablauf einfügendes Verfahren und deshalb kostengünstig aufgebracht werden kann und eine Membran mit gleichbleibender Porengröße liefert.

Diese Aufgabe wird durch eine Paste für eine poröse Polymermembran gemäß Anspruch 1 gelöst. Vorteilhafte Ausgestaltungen ergeben sich aus den Ansprüchen 2 bis 18.

Im folgenden wird die Erfindung anhand der Figuren erläutert, wobei
Figur 1 schematisch den Aufbau eines Teststreifens mit der erfindungsgemäßen Membran zeigt,
Figur 2 eine rheologische Charakteristik der erfindungsgemäßen Paste zeigt,
Figur 3a eine elektronenmikroskopische Aufnahme einer Polymermembran mit unzureichend ausgebildeter Porenstruktur zeigt,
Figur 3b eine elektronenmikroskopische Aufnahme der erfindungsgemäßen Polymermembran mit gut ausgebildeter Porenstruktur zeigt,
Figur 4 die Messergebnisse zweier Biosensoren, wobei einer von ihnen mit einer erfindungsgemäßen Membran versehen ist, bei ansteigenden Hämatokritwerten im Vergleich zeigt,
die Figuren 5a bis 5d die klinische Performanz vierer Blutglucosesensoren im Vergleich zeigen.

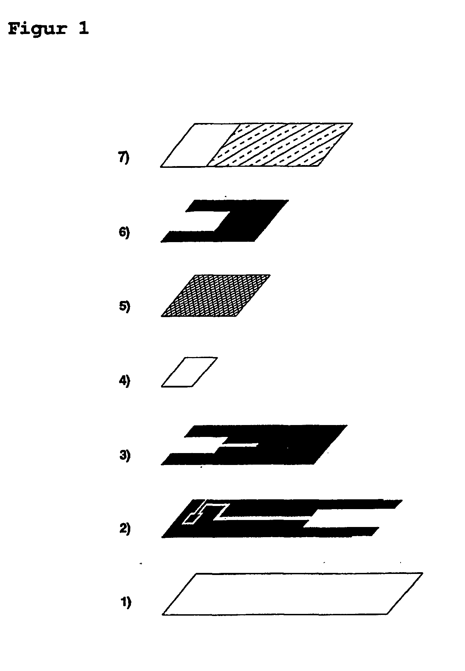

In Figur 1 ist der Aufbau eines Teststreifens mit der erfindungsgemäßen Polymermembran dargestellt. Auf einem Polyester-Trägermaterial 1 befindet sich eine Elektrodenanordnung 2 in Form einer Kohlenstoffschicht, die wiederum teilweise von einer Isolierung 3 abgedeckt wird. Eine Enzym- und Mediatorschicht 4 ist auf dem Bereich der Elektrodenschicht angeordnet, der von der Isolierung freigelassen wird. Im Falle eines Blutglucosesensors enthält diese Schicht beispielsweise das Enzym Glucoseoxidase und den Mediator Fe³⁺. Die erfindungsgemäße Polymermembran 5 ist über der Enzym- und Mediatorschicht 4 angeordnet. Das ganze wird durch eine Klebeschicht 6 und eine Deckelfolie 7 abgedeckt.

Bei der Massenherstellung von Biosensoren wird für das Aufdrucken der verschiedenen Schichten, wie Elektroden-, Isolier- und Enzymschichten, das Siebdruckverfahren angewendet. Ein System unter Anwendung der Siebdrucktechnik wird in der US 5,607,566 beschrieben. Dort wird die Technik bei der chargenweisen Fertigung der Kontakte und Polymermembranen von ionenselektiven Festkörpersensoren angewandt. Die Polymermembranpastenzusammensetzungen enthalten eine Polyurethan und hydroxyliertes Polyvinylchlorid enthaltende Masse und eine auf Silikon basierende Masse in geeigneten Lösungsmittelsystemen zur Bereitstellung von siebdruckfähigen Pasten mit geeigneter Viskosität und Thixotropie. Die vorliegende Erfindung stellt eine Membran zur Verfügung, die mit der gleichen Technik aufgebracht werden kann. Das hat einerseits den Vorteil, dass für das Aufdrucken der Membran und damit während des gesamten Herstellungsprozesses des Sensors die gleiche Siebdruckvorrichtung verwendet werden kann, was bei der Massenproduktion enorme ökonomische Vorteile mit sich bringt. Zum anderen kann durch das Siebdruckverfahren reproduzierbar eine Membran gleichmäßiger Dicke und Porehgröße hergestellt werden, was mit den anderen Methoden, wie Aufspinnen, Eintauchen oder Aufsprühen nicht gewährleistet ist.

Damit die zur Herstellung der Polymermembran verwendete Paste durch Siebdruck aufgebracht werden kann, müssen das oder die darin enthaltenen Lösungsmittel für das Polymere einen möglichst hohen Siedepunkt (über 100 °C) aufweisen, um das vorzeitige Trocknen des Materials in der Druckmaschine zu vermeiden. Außerdem enthält die Paste ein Nichtlösungsmittel für das Polymere, das als Porenbildner fungiert und einen höheren Siedepunkt als das oder die verwendeten Lösungsmittel aufweist.

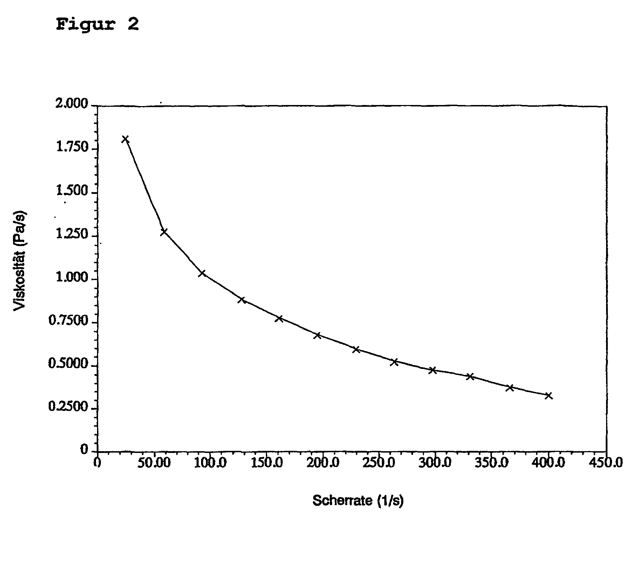

Zudem muss die Paste eine geeignete Viskosität (30.000 - 50.000 cpi) besitzen, um einen gleichmäßigen Fluss durch das Sieb während des Aufdruckens zu gewährleisten. Bevorzugt verringert sich die Viskosität der Paste bei Einwirkung von Scherkräften, wie in der rheologischen Charakteristik in Figur 2 dargestellt.

Als Polymeres wird in der erfindungsgemäßen Paste bevorzugt Zelluloseacetat (50 kDa) verwendet. Es ist bevorzugt mit einem Anteil von etwa 8 Gew.-% in der siebdruckfähigen Paste enthalten. Außerdem kann als weiteres Polymeres Zellulosenitrat mit einem Anteil von bis zu 10 Gew.-% enthalten sein.

Als Lösungsmittel für das Polymere können beispielsweise 1,4-Dioxan (Siedepunkt 102 °C) und/oder 4-Hydroxymethylpentanon (Siedepunkt 165 °C) verwendet werden. Eine bevorzugte Zusammensetzung enthält 0 - 20 Gew.-%, bevorzugter 20 Gew.-%, 1,4-Dioxan und 0 - 70 Gew.-%, bevorzugter 56 Gew.-%, 4-Hydroxymethylpentanon, wobei das 4-Hydroxymethylpentanon alternativ durch Ethylacetat oder Ethylenglycoldiacetat ersetzt sein kann.

Es stellte sich heraus, dass als Porenbildner für die siebdruckfähige Membranpaste langkettige Alkohole mit einem Siedepunkt von > 150 °C geeignet sind; bevorzugt werden n-Octanol, welches einen Siedepunkt von 196 °C aufweist, und/oder 2-Methyl-2,4-pentandiol (MPD), welches einen Siedepunkt von 197 °C aufweist, verwendet.

Bei Verwendung von 2-Metyl-2,4-pentandiol (MPD) als Porenbildner ist die Paste etwas toleranter gegenüber dem Abdampfen von Dioxan. Auch bleibt das Zelluloseacetat länger in Lösung, wodurch sich der Zeitraum verlängert, in dem die Paste in druckfähigem Zustand bleibt. Diese verlängerte "Potzeit" ermöglicht die Produktion größerer Chargen mit gleichbleibender Qualität.

Der Porenbildner sollte mit einem Anteil von 5 - 20 Gew.-%, bevorzugt 12 - 15 Gew.-%, enthalten sein.

Als Viskositätsmodifizierer werden beispielsweise hydrophile Kiesel-Xerogele oder äquivalente "Fumed Silicas", Bentonite Clay, Natrosol oder Carbon Black verwendet. Sie sollten mit einem Anteil von 1 bis 10 Gew.-% der siebdruckfähigen Paste zugesetzt werden. Bevorzugt werden hydrophile Cab-O-Sile (Handelsbezeichnung für Kiesel-Xerogele, vertrieben durch das Unternehmen Cabot), wie Cab-O-Sil M5, Cab-O-Sil H5, Cab-O-Sil LM150, Cab-O-Sil LM130, mit einem Anteil von 4 Gew.-% verwendet.

Außerdem können weitere Zusatzstoffe, wie Tween 20, Triton X, Silvet 7600 oder 7280, Laurylsulfat (SDS), andere Detergenzien sowie Polyole, wie Glycerol, oder hydrophile Polymere, wie Polyvinylpyrolidon (PVP) bzw. Vinylpyrolidon-Vinylacetat-Copolymere (PVP/VA) der erfindungsgemäßen Paste zugesetzt werden.

Der Zusatz eines oder mehrerer dieser Zusatzstoffe ist nicht obligatorisch für die Herstellung der Membran; es zeigte sich jedoch, dass sie das Benetzen der Membran verbessern und die Sensorantwort beschleunigen können. Bevorzugt wird PVP/VA oder PVP mit einem Anteil von 0,1 Gew.-% in der siebdruckfähigen Paste verwendet.

Außerdem kann die Zugabe der Additive Bioterge, Polyethylenimin, BSA, Dextran, Dicyclohexylphthalat, Gelatine, Sucrose und/oder Biuret die Separation von Erythrozyten und Plasma verbessern.

Zudem ist es möglich, der Zelluloseacetatpaste bereits Enzym, beispielsweise Glucoseoxidase, zuzusetzen, so dass im Herstellungsprozess des Biosensors das Aufdrucken der Enzymschicht entfallen kann.

Nach dem Aufbringen einer gleichmäßigen Schicht der Druckpaste auf ein geeignetes Substrat bildet sich die Membran im Trocknungsprozess. Es bildet sich eine poröse Schicht und kein geschlossener Film, da die verwendeten Lösungsmittel einen niedrigeren Siedepunkt besitzen als der Porenbildner; entsprechend schnell verdampfen die Lösungsmittel und das Zelluloseacetat-Polymere fällt in dem verbliebenen Film des Porenbildners aus.

In Verbindung mit einem Biosensor darf in dem Trocknungsprozess jedoch nicht eine beliebig hohe Temperatur verwendet werden, da bei zu hohen Temperaturen die verwendeten Enzyme / Proteine denaturiert werden. In Verbindung mit einem Biosensor zur Bestimmung von Glucose im Vollblut wurden mit einer Trocknungstemperatur von etwa 70 °C die besten Ergebnisse erzielt. Entsprechend sollten die Siedepunkte der verwendeten Lösungsmittel und Porenbildner ausgewählt werden.

Einen entscheidenden Faktor für die Porenbildung spielt der verwendete Viskositätsmodifizierer, der zusammen mit dem Porenbildner ein Gel bildet, um die Polymerstruktur zu stabilisieren. Bei den verwendeten Substanzen entsteht das Gel durch die Wechselwirkung zwischen den OH-Gruppen des Kiesel-Xerogels und dem langkettingen Alkohol (z.B. Octanol). Die Menge und die Verteilung des Gels, das während des Trocknungsprozesses entsteht, entscheidet schließlich über die Größe und die Form der sich ausbildenden Poren.

Ohne Zugabe eines Viskositätsmodifizierers bildet sich aus dem Lösungsmittel und dem Porenbildner eine Emulsion, da der Porenbildner alleine nicht in der Lage ist, das Polymerskelett zu stabilisieren. Als Resultat erhält man einen weißen, glatten und unstrukturierten Film mit eingeschlossenem Porenbildner, der keinen lateralen Flüssigkeitstransport erlaubt. Im Vergleich dazu erhält man einen klaren Film, wenn kein Porenbildner in der Paste verwendet wird.

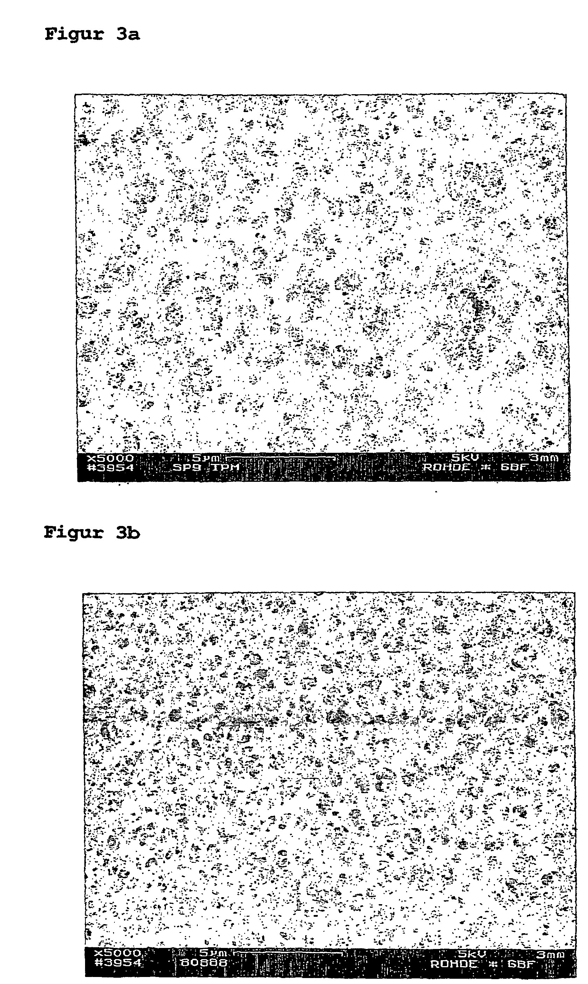

Bei Verwendung zu geringer Mengen an Viskositätsmodifizierer (< 1 Gew.-%) erhält man eine Membran mit nur unzureichend ausgebildeter Porenstruktur, wie sie in Figur 3a wiedergegeben ist.

Da die verschiedenen geeigneten Viskositätsmodifizierer unterschiedliche Oberflächeneigenschaften aufweisen, kann der Viskositätsmodifizierer in Abhängigkeit der gewünschten Membran oder des gewünschten Biosensors ausgewählt werden. Beispielsweise wird das Cab-O-Sil H5 bei hoher mechanischer Belastung, z. B. bei langen Druckzeiten oder beim Drucken sehr dünner Schichten mit hohem Rakeldruck, "zerrieben". Die Oberfläche zeigt dann mikroskopisch scharfe Bruchkanten, die zur Lysierung der roten Blutzellen führen können.

Für einen Blutzuckersensor ist dies eine unerwünschte Eigenschaft, da dadurch der Grundstrom des Sensors erhöht wird. Andererseits kann dieser Effekt optimiert und das Plasma von Zellen direkt im Sensor für die elektrochemische Detektion genutzt werden. Ein praktisches Beispiel wäre die Bestimmung von Hämoglobin in Erythrozyten. In diesem Fall reagiert der Mediator des Biosensors, z. B. Kaliumhexacyanoferrat(III), mit der Fe(II)-Gruppe des Hämoglobins, wodurch Kaliumhexazyanoferrat(II) erzeugt wird, welches direkt an der Elektrode des Biosensors bestimmt werden kann. Ein Enzym, wie im Fall der Glukosebestimmung, ist hier nicht notwendig, da der Mediator direkt mit dem Hämoglobin reagiert. In der Praxis kann so die Bestimmung des Hämatokritwerts eines Patienten mit ähnlichen Messgeräten wie bei der Blutzuckerkontrolle durchgeführt werden, wodurch sich die zeitaufwendige Verwendung von Kapillarröhrchen und Zentrifuge erübrigt.

Das Cab-O-Sil LM 150 besteht aus kleineren Partikeln als H5, die daher stabiler sind und nicht durch den mechanischen Stress beim Druckprozess beschädigt werden. Dieser Viskositätsmodifizierer ist daher für die Herstellung einer Membran für Blutzuckersensoren bestens geeignet.

Entsprechend der obigen Ausführung ist die Differenz der Siedepunkte zwischen Lösungsmittel und Porenbildner neben der Stabilisierung des Polymerskeletts durch den Viskositätsmodifizierer von Bedeutung für die Ausbildung einer geeigneten Membran. Hierbei sollte die Differenz etwa 30 °C betragen, damit im Trocknungsprozess ein Film ausgebildet wird, der eine ausreichend hohe Konzentration an Porenbildner enthält, in dem das Membranpolymer ausfallen kann. Bei geringeren Siedepunktsdifferenzen beginnt der Porenbildner zu verdampfen, bevor ein kritisches Verhältnis zwischen Lösungsmittel und Porenbildner erreicht wird, welches das Ausfallen des Membranpolymeren bewirkt.

Nach Aufdrucken der siebdruckfähigen Paste mit der zuvor beschriebenen Zusammensetzung und Verdampfen des Lösungsmittels bildet sich durch Ablagerung der Zelluloseester eine Membran mit einer durchschnittlichen Porengröße von 0,1 bis 2 µm, wobei die Porengröße durch die verwendete Menge des langkettigen Alkohols beeinflusst werden kann. Eine elektronenmikroskopische Aufnahme der Membran ist in Figur 3b wiedergegeben. Da die Erythrozyten eine durchschnittliche Größe von 8 bis 10 µm aufweisen, werden sie durch die Membran von der Enzymschicht zurückgehalten, während das Plasma ungehindert passieren kann. Zusätzlich trägt die Membran zur mechanischen Stabilität der Enzymschicht bei und verhindert, dass sich das Enzym beim Auftragen der Blutprobe von der Elektrode ablöst und dann nicht mehr für die elektrochemische Reaktion zur Verfügung steht.

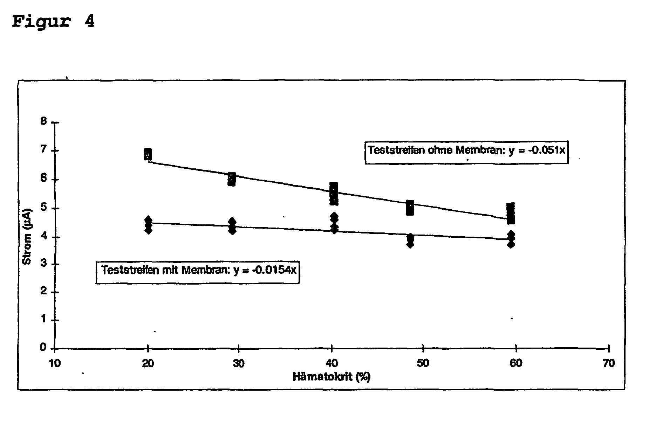

In Figur 4 wird anhand einer Messreihe verdeutlicht, dass bei konstanter Glucosekonzentration der mit einer erfindungsgemäßen Membran versehene Teststreifen im Gegensatz zu einem Teststreifen ohne Membran bei steigenden Hämatokritwerten gleichbleibende Ergebnisse liefert, während bei dem Teststreifen ohne Membran die Antwort bei steigender Erythrozytenkonzentration abnimmt. Aufgrund der erhöhten Diffusionsbarriere zwischen der Enzymschicht und der Blutprobe ist die Antwort bei dem Sensor mit Membran insgesamt etwas verringert.

Die Erfindung wird anhand der folgenden Beispiele verdeutlicht.

### Herstellung der Druckpaste:

Entsprechend den in den folgenden Beispielen angegebenen Mengenverhältnissen wird eine Mischung aus dem Lösungsmittel (z.B. Hydroxymethylpenanon, Dioxan) und dem Porenbildner (z.B. Octanol, MPD) hergestellt, damit eine gleichmäßige Verteilung beider Substanzen gewährleistet ist. Im nächsten Schritt werden alle Additive (z.B. PVP/VA) zugefügt und, falls erforderlich, mit Hilfe von Ultraschall aufgelöst. Anschließend wird das Membranpolymer (Zelluloseactat 50 kDa) zügig unter das zuvor hergestellte Lösungsmittel gemischt, bis eine gleichmäßige Suspension entsteht. Diese Suspension rollt für 48 h in einem verschlossenen Behälter bis ein klares Gel entsteht, dem der Viskositätsmodifizierer (z. B. Cab-O-Sil) zugesetzt werden kann. Die fertige Druckpaste rollt für weiter 24 h, um eine gleichmäßige Verteilung des Viskositätsmodifizierers zu gewährleisten.

### Beispiel 1

| Polymer(e): | |
|---|---|
| Zelluloseacetat (Mw 30000) | 7,5 Gew.-% |

| Lösungsmittel: | |
|---|---|
| Ethylenglycoldiacetat (Sp 186 °C) | 65,5 Gew.-% |

| Porenbildner: | |
|---|---|
| n-Decanol (Sp 231°C) | 25,0 Gew.-% |

| Viskositätsmodifizierer: | |
|---|---|
| Cab-O-Sil M5 | 2,0 Gew.-% |

### Beispiel 2

| Polymer(e): | |
|---|---|
| Zelluloseacetat (Mw 50000) | 8,0 Gew.-% |

| Lösungsmittel: | |
|---|---|
| 1,4-Dioxan (Sp 102 °C) | 35,0 Gew.-% |
| Ethylacetat (Sp 154 °C) | 35,0 Gew.-% |

| Porenbildner: | |
|---|---|
| n-Octanol (Sp 196 °C) | 18,0 Gew.-% |

| Viskositätsmodifizierer: | |
|---|---|
| Cab-O-Sil M5 | 4,0 Gew.-% |

### Beispiel 3

| Polymer(e): | |
|---|---|
| Zelluloseacetat (Mw 50.000) | 8,0 Gew.-% |

| Lösungsmittel: | |
|---|---|
| 1,4-Dioxan (Sp 102 °C) | 20,0 Gew.-% |
| 4-Hydroxymethylpentanon (Sp 165 °C) | 56,0 Gew.-% |

| Porenbildner: | |
|---|---|
| n-Octanol (Sp 196 °C) | 12,0 Gew.-% |

| Viskositätsmodifizierer: | |
|---|---|
| Cab-O-Sil M5 | 4,0 Gew.-% |

| Additive: | |
|---|---|
| PVP/VA | 0,1 Gew.-% |

### Beispiel 4

| Polymer(e): | |
|---|---|
| Zelluloseacetat (Mw 50.000) | 7,4 Gew.-% |

| Lösungsmittel: | |
|---|---|
| 1,4-Dioxan (Sp 102 °C) | 18,5 Gew.-% |
| 4-Hydroxymethylpentanon (Sp 165 °C) | 55,6 Gew.-% |

| Porenbildner: | |
|---|---|
| 2-Methyl-2,4-pentandiol | 14,8 Gew.-% |

| Viskositätsmodifizierer: | |
|---|---|
| Cab-O-Sil M5 | 3,7 Gew.-% |

| Additive: | |
|---|---|
| PVP/VA | 0,1 Gew.-% |

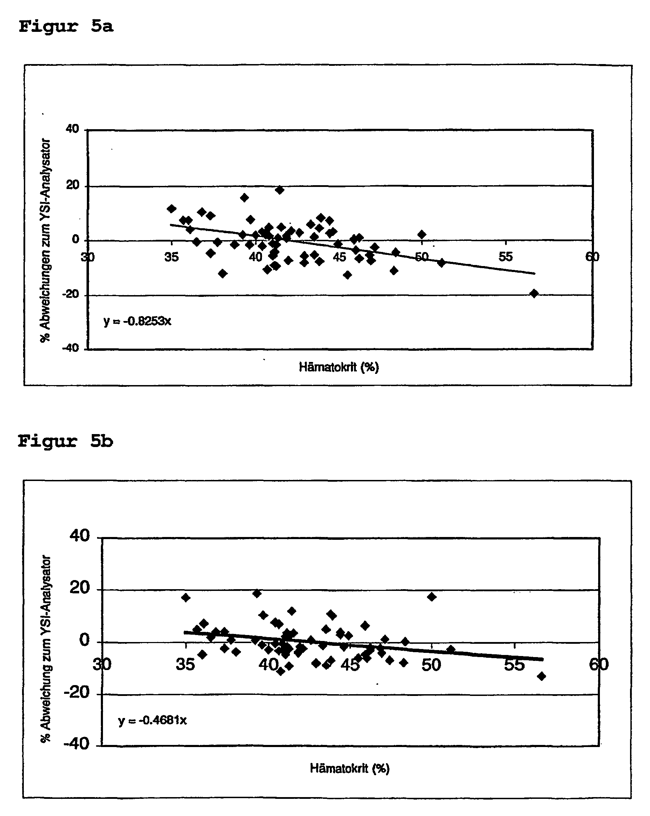

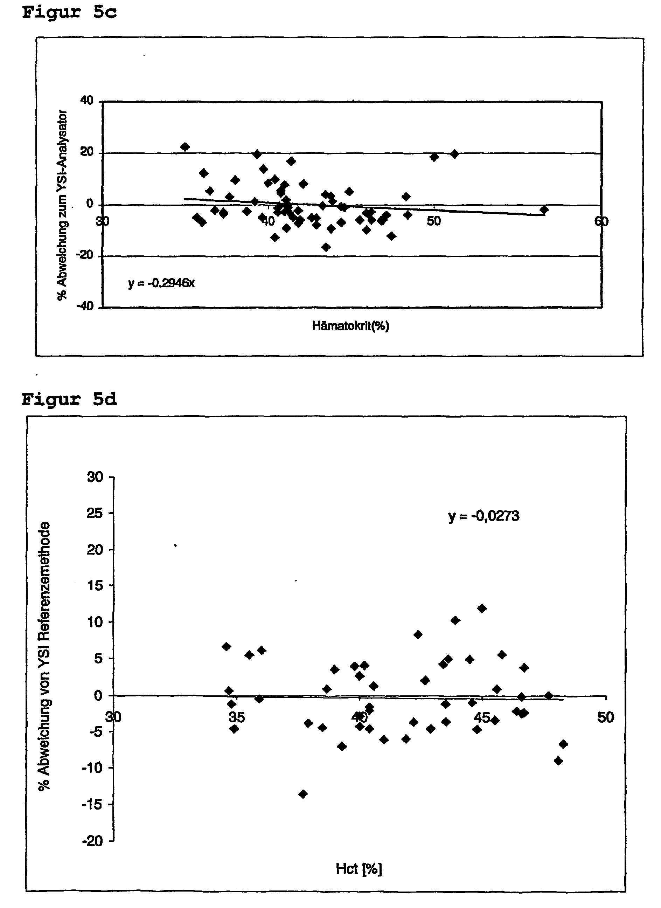

Figur 5 zeigt die klinische Performanz von Blutglucosesensoren
a) ohne Polymermembran
b) mit Polymermembran (Zusammensetzung gemäß Beispiel 2)
c) mit Polymermembran (Zusammensetzung gemäß Beispiel 3)
d) mit Polymermembran (Zusammensetzung gemäß Beispiel 4).

Bei den vergleichenden klinischen Untersuchungen wurden die Messergebnisse der unterschiedlichen Sensortypen mit den Messergebnissen der Referenzmethode (YSI Model 2300 Stat Plus) verglichen und die prozentuale Abweichung über die Hämatokritwerte der einzelnen Blutproben aufgetragen. Im Idealfall ergibt sich eine Messsgerade horizontal zur x-Achse. Die Steigung dieser Messgeraden, welche in Tabelle 1 wiedergegeben ist, gibt Aufschluss über die Hämatokritinterferenz des verwendeten Sensorsystems.

**Tabelle 1**

| | Steigung der Messgeraden | Steigung in % |
|---|---|---|
| Typ 1 (keine Membran) | -0,8253 | 100 % |
| Typ 2 (Membran aus Beispiel 2) | -0,4681 | 56 % |
| Typ 3 (Membran aus Beispiel 3) | -0,2946 | 35 % |
| Typ 4 (Membran aus Beispiel 4) | -0,0273 | 3,3 % |

Die Daten lassen eindeutig die überlegene Performanz des Sensorsystems mit der bevorzugten Membran (Zusammensetzung gemäß Beispiel 4) erkennen. Diese Verbesserung wird durch die Separation von Vollblut und Plasma unmittelbar vor der Elektrode erreicht, da sich die Nernstsche Diffusionsschicht vor der Elektrode nicht mehr in den Bereich mit Erythrozyten ausdehnt und daher auch nicht mehr durch unterschiedliche Hämatokritwerte beeinflusst werden kann.

In den folgenden Vergleichsbeispielen werden Druckpasten beschrieben, bei denen keine geeignete Abstimmung zwischen dem Porenbildner, den Lösungsmitteln und dem Viskositätsmodifizierer besteht.

### Vergleichsbeispiel 1

| Polymer(e): | |
|---|---|
| Zelluloseacetat (Mw 50.000) | 8,0 Gew.-% |

| Lösungsmittel: | |
|---|---|
| Ethylenglycoldiacetat (Sp 186 °C) | 76,0 Gew.-% |

| Porenbildner: | |
|---|---|
| n-Octanol (Sp 196 °C) | 12,0 Gew.-% |

| Viskositätsmodifizierer: | |
|---|---|
| Cab-O-Sil M5 (hydrophil) | 4,0 Gew.-% |

| Additive: | |
|---|---|
| PVP/VA | 0,1 Gew.-% |

### Vergleichsbeispiel 2

| Polymer(e): | |
|---|---|
| Zelluloseacetat (Mw 50.000) | 8,0 Gew.-% |

| Lösungsmittel: | |
|---|---|
| 1,4-Dioxan (Sp 102 °C) | 20,0 Gew.-% |
| 4-Hydroxymethylpentanon (Sp 165 °C) | 56,0 Gew.-% |

| Porenbildner: | |
|---|---|
| n-Octanol (Sp 196 °C) | 12,0 Gew.-% |

| Viskositätsmodifizierer: | |
|---|---|
| Cab-O-Sil TS720 (hydrophob) | 4,0 Gew.-% |

| Additive: | |
|---|---|
| PVP/VA | 0,1 Gew.-% |

### Vergleichsbeispiel 3

| Polymer(e): | |
|---|---|
| Zelluloseacetatpropionat (Mw 75.000) | 8,0 Gew.-% |

| Lösungsmittel: | |
|---|---|
| 1,4-Dioxan (Sp 102 °C) | 20,0 Gew.-% |
| 4-Hydroxymethylpentanon (Sp 165 °C) | 56,0 Gew.-% |

| Porenbildner: | |
|---|---|
| n-Octanol (Sp 196 °C) | 12,0 Gew.-% |

| Viskositätsmodifizierer: | |
|---|---|
| Cab-O-Sil M5 (hydrophil) | 4,0 Gew.-% |

| Additive: | |
|---|---|
| PVP/VA | 0,1 Gew.-% |

In Vergleichsbeispiel 1 wird aufgrund der zu geringen Differenz der Siedepunkte von in der Druckpaste verwendetem Lösungsmittel (Ethylenglycoldiacetat) und Porenbildner (n-Octanol) keine poröse Membran ausgebildet. Wird hingegen n-Decanol als Porenbildner verwendet (wie in Beispiel 1 beschrieben) erhält man eine poröse Membran nach dem Trocknungsprozess, da der Siedepunkt zwischen dem Lösungsmittel und dem Porenbildner ausreichend groß ist.

In Vergleichsbeispiel 2 findet aufgrund der Verwendung von hydrophobem Cab-O-Sil, welches nicht in der Lage ist, mit den OH-Gruppen des Porenbildners zu reagieren, nur eine unzureichende Gelbildung zwischen dem Porenbildner und dem Viskositätsmodifizierer und somit keine ausreichende Stabilisierung des Polymerskeletts statt. Dadurch wird die Bildung einer porösen Membran verhindert.

Auch in Vergleichsbeispiel 3, wo das verwendete Polymere (Zelluloseacetatpropionat) eine zu hohe Löslichkeit in dem Porenbildner aufweist, wird keine poröse Membran ausgebildet.

## Patentansprüche

1. Siebdruckfähige Paste zur Herstellung einer porösen Polymermembran, enthaltend wenigstens ein Polymeres, ein oder mehrere Lösungsmittel für das Polymere mit einem Siedepunkt von > 100 °C, ein oder mehrere Nichtlösungsmittel (Porenbildner) für das Polymere mit einem höheren Siedepunkt als das/die Lösungsmittel und einen hydrophilen Viskositätsmodifizierer.

2. Siebdruckfähige Paste gemäß Anspruch 1, **dadurch gekennzeichnet, dass** die Differenz der Siedepunkte von Lösungsmittel und Porenbildner wenigstens 30 °C beträgt.

3. Siebdruckfähige Paste gemäß Anspruch 1 oder 2, **dadurch gekennzeichnet, dass** die Paste als Polymeres Zelluloseacetat enthält.

4. Siebdruckfähige Paste nach Anspruch 3, **dadurch gekennzeichnet, dass** die Paste als Lösungsmittel 1,4-Dioxan und/oder 4-Hydroxymethylpentanon und/oder Ethylacetat enthält.

5. Siebdruckfähige Paste nach wenigstens einem der Ansprüche 1 bis 4, **dadurch gekennzeichnet, dass** die Paste als Porenbildner einen langkettigen Alkohol enthält.

6. Siebdruckfähige Paste nach Anspruch 5, **dadurch gekennzeichnet, dass** die Paste als Porenbildner n-Octanol und /oder 2-Methyl-2,4-pentandiol enthält.

7. Siebdruckfähige Paste nach Anspruch 6, **dadurch gekennzeichnet, dass** n-Octanol und/oder 2-Methyl-2,4-pentandiol mit einem Anteil von 5 - 20 Gew.-% enthalten ist.

8. Siebdruckfähige Paste nach wenigstens einem der vorhergehenden Ansprüche, **dadurch gekennzeichnet, dass** die Paste als Viskositätsmodifizierer hydrophiles Kiesel-Xerogel enthält.

9. Siebdruckfähige Paste gemäß Anspruch 8, **dadurch gekennzeichnet, dass** das Kiesel-Xerogel mit einem Anteil von 1 - 10 Gew.-% enthalten ist.

10. Siebdruckfähige Paste nach wenigstens einem der vorhergehenden Ansprüche, **dadurch gekennzeichnet, dass** die Paste außerdem Vinylpyrolidon-Vinylacetat-Copolymere (PVP/VA) und/oder Polyvinylpyrolidon (PVP) enthält.

11. Siebdruckfähige Paste nach Anspruch 10, **dadurch gekennzeichnet, dass** das PVP/VA bzw. PVP mit einem Anteil von 0,1 Gew.-% enthalten ist.

12. Siebdruckfähige Paste nach wenigstens einem der vorhergehenden Ansprüche, **dadurch gekennzeichnet, dass** die Paste außerdem ein oder mehrere Enzyme enthält.

13. Verfahren zur Herstellung einer siebdruckfähigen Paste, in dem man
eine Mischung aus einem oder mehreren Lösungsmittel(n) für ein Polymeres und einem oder mehreren Nichtlösungsmittel(n) für ein Polymeres (Porenbildner) herstellt,
das Polymere bis zum Entstehen einer gleichmäßigen Suspension untermischt,
die Suspension bis zum Entstehen eines klaren Gels rollt,
einen hydrophilen Viskositätsmodifizierer zugibt und
das ganze bis zur gleichmäßigen Verteilung des Viskositätsmodifiziereres rollt, bei dem das/die Lösungsmittel einen Siedepunkt von >100°C aufweist/aufweisen und das/die Nichtlösungsmittel einen höheren Siedepunkt als das/die Lösungsmittel aufweist/aufweisen.

14. Verwendung der Paste nach wenigstens einem der Ansprüche 1 bis 12 zur Herstellung einer porösen Polymermembran.

15. Verwendung gemäß Anspruch 14, wobei die Polymermembran in einen Biosensor-Teststreifen eingebracht wird.

16. Verwendung gemäß Anspruch 15, **dadurch gekennzeichnet, dass** der Biosensor zur Messung der Blutglucosekonzentration ausgebildet ist.

17. Verwendung gemäß Anspruch 15, **dadurch gekennzeichnet, dass** der Biosensor zur Bestimmung des Hämatokritwerts ausgebildet ist.

18. Poröse Polymermembran, hergestellt aus der siebdruckfähigen Paste gemäß wenigstens einem der Ansprüche 1 bis 12.

## Claims

1. Screen-printable paste for producing a porous polymer membrane, comprising at least one polymer, one or more solvents for the polymer with a boiling point of > 100°C, one or more nonsolvents (pore formers) for the polymer with a higher boiling point than the solvent(s) and a hydrophilic viscosity modifier.

2. Screen-printable paste according to Claim 1, **characterized in that** the difference of the boiling points of solvent and pore former is at least 30°C.

3. Screen-printable paste according to Claim 1 or 2, **characterized in that** the paste comprises cellulose acetate as polymer.

4. Screen-printable paste according to Claim 3, **characterized in that** the paste comprises 1,4-dioxane and/or 4-hydroxymethylpentanone and/or ethyl acetate as solvent.

5. Screen-printable paste according to at least one of Claims 1 to 4, **characterized in that** the paste comprises a long-chain alcohol as pore former.

6. Screen-printable paste according to Claim 5, **characterized in that** the paste comprises n-octanol and/or 2-methyl-2,4-pentanediol as pore former.

7. Screen-printable paste according to Claim 6, **characterized in that** n-octanol and/or 2-methyl-2,4-pentanediol is present in a proportion of 5-20% by weight.

8. Screen-printable paste according to at least one of the preceding claims, **characterized in that** the paste comprises hydrophilic silica xerogel as viscosity modifier.

9. Screen-printable paste according to Claim 8, **characterized in that** the silica xerogel is present in a proportion of 1-10% by weight.

10. Screen-printable paste according to at least one of the preceding claims, **characterized in that** the paste additionally comprises vinylpyrolidone/vinyl acetate copolymer (PVP/VA) and/or polyvinylpyrolidone (PVP).

11. Screen-printable paste according to Claim 10, **characterized in that** the PVP/VA or PVP is present in a proportion of 0.1% by weight.

12. Screen-printable paste according to at least one of the preceding claims, **characterized in that** the paste additionally comprises one or more enzymes.

13. Method for producing a screen-printable paste, by
producing a mixture of one or more solvent(s) for a polymer and one or more nonsolvent(s) for a polymer (pore former),
mixing in the polymer until a uniform suspension results,
rolling the suspension until a clear gel results,
adding a hydrophilic viscosity modifier, and
rolling the mixture until the viscosity modifier is uniformly distributed, in which method the solvent(s) has/have a boiling point of > 100°C and the nonsolvent(s) has/have a higher boiling point than the solvent(s).

14. Use of the paste according to at least one of Claims 1 to 12 for producing a porous polymer membrane.

15. Use according to Claim 14, where the polymer membrane is introduced into a biosensor test strip.

16. Use according to Claim 15, **characterized in that** the biosensor is designed for measuring the blood glucose concentration.

17. Use according to Claim 15, **characterized in that** the biosensor is designed for determining the value of the haematocrit.

18. Porous polymer membrane produced from the screen-printable paste according to at least one of Claims 1 to 12.

## Revendications

1. Pâte pouvant être imprimée par sérigraphie utilisée pour produire une membrane polymère poreuse, contenant au moins un polymère, un ou plusieurs solvants pour le polymère avec un point d'ébullition supérieur à 100°C, un ou plusieurs non-solvants (formateurs de pores) pour le polymère avec un point d'ébullition plus élevé que celui du/des solvants et un modificateur de viscosité hydrophile.

2. Pâte pouvant être imprimée par sérigraphie selon la revendication 1, **caractérisée en ce que** la différence entre les points d'ébullition des solvants et ceux des formateurs de pores s'élève au moins à 30°C.

3. Pâte pouvant être imprimée par sérigraphie selon la revendication 1 ou 2, **caractérisée en ce que** la pâte contient de l'acétate de cellulose comme polymère.

4. Pâte pouvant être imprimée par sérigraphie selon la revendication 3, **caractérisée en ce que** la pâte contient du 1,4-dioxane et/ou de la 4-hydroxyméthylpentanone et/ou de l'acétate d'éthyle comme solvant.

5. Pâte pouvant être imprimée par sérigraphie selon au moins l'une des revendications 1 à 4, **caractérisée en ce que** la pâte contient un alcool à chaîne longue comme formateur de pores.

6. Pâte pouvant être imprimée par sérigraphie selon la revendication 5, **caractérisée en ce que** la pâte contient du n-octanol et/ou du 2-méthyl-2,4-pentanediol comme formateurs de pores.

7. Pâte pouvant être imprimée par sérigraphie selon la revendication 6, **caractérisée en ce que** le n-octanol et/ou le 2-méthyl-2,4-pentanediol est contenu dans une proportion de 5 à 20 % en poids.

8. Pâte pouvant être imprimée par sérigraphie selon au moins l'une des revendications précédentes, **caractérisée en ce que** la pâte contient un xérogel silicique hydrophile comme modificateur de viscosité.

9. Pâte pouvant être imprimée par sérigraphie selon la revendication 8, **caractérisée en ce que** le xérogel silicique est contenu dans une proportion de 1 à 10 % en poids.

10. Pâte pouvant être imprimée par sérigraphie selon au moins l'une des revendications précédentes, **caractérisée en ce que** la pâte contient en outre un copolymère vinylpyrrolydone-acétate de vinyle (PVP/VA) et/ou de la polyvinylpyrrolidone (PVP).

11. Pâte pouvant être imprimée par sérigraphie selon la revendication 10, **caractérisée en ce que** le PVP/VA et/ou la PVP est contenu dans une proportion de 0,1 % en poids.

12. Pâte pouvant être imprimée par sérigraphie selon au moins l'une des revendications précédentes, caractérisée en ce la pâte contient en outre une ou plusieurs enzymes.

13. Procédé pour préparer une pâte pouvant être imprimée par sérigraphie, dans lequel
■ on prépare un mélange à partir d'un ou de plusieurs solvant(s) pour un polymère et d'un ou de plusieurs non-solvant(s) (formateurs de pores),
■ on y mélange le polymère jusqu'à ce qu'on obtienne une suspension équilibrée,
■ on enroule la suspension jusqu'à ce qu'on obtienne un gel clair,
■ on ajoute un modificateur de viscosité et
■ on enroule le tout jusqu'à de que le modificateur de viscosité se répartisse de façon équilibrée, dans lequel le/les solvants présentent un point d'ébullition supérieur à 100°C et le/les non-solvants présentent un point d'ébullition supérieur à celui des solvants.

14. Utilisation de la pâte selon au moins l'une des revendications 1 à 12 pour produire une membrane polymère poreuse.

15. Utilisation selon la revendication 14, la membrane polymère étant présente dans la bandelette réactive d'un biocapteur.

16. Utilisation selon la revendication 15, **caractérisée en ce que** le biocapteur est conçu pour mesurer la concentration de glucose dans le sang.

17. Utilisation selon la revendication 15, **caractérisée en ce que** le biocapteur est conçu pour déterminer le taux d'hématocrites.

18. Membrane polymère poreuse produite à partir d'une pâte pouvant être imprimée par sérigraphie selon au moins l'une quelconque des revendications 1 à 12.
